# EUROPEAN PATENT APPLICATION

(11) **EP 4 692 543 A2**
(43) Date of publication of application: **11.02.2026**
(21) Application number: 25202552.3
(22) Date of filing: 01.06.2020
(51) Int. Cl.: F04B 49/06

(54) **PERISTALTIC PUMP-BASED APPARATUS AND METHOD FOR THE CONTROLLED DISPENSING OF FLUIDS**

(30) Priority: 03.06.2019 US 201916430383
(62) Divisional of application: 20818477.0
(71) Applicant: VANRX Pharmasystems Inc., Burnaby, BC V5J 5J2 (CA)
(72) Inventor: CICHY, Marcin, Surrey, V3S 3B8 (CA); GUERRERO, Carlos Alberto Diaz, Burnaby, V3N 5C4 (CA)
(74) Representative: Pfundner, Benjamin Patrick

(57) **Abstract**

The present invention involves a rotary peristaltic pump and associated method employing the relaxation of pressure on flexible tubing between the rotor and stator of the pump to restore the rotor to a start angle while a valve on the output of the pump is closed to avoid progress of fluid through the system. Three different implementations of the pump and method are presented including reciprocating the stator, reciprocating the rotor, and retracting the idler rollers of the rotor into the rotor to relieve the pressure on the flexible tubing. A controller ensures the appropriate switching and timing of the valve and rotor of the pump. The pump and associated method are directed to the precision dispensing of pharmaceutical fluids into containers, including containers held in container nests.

## Description

### BACKGROUND OF THE INVENTION

### Field of the Invention

This present invention relates to the medical field and more particularly to those in international patent classification A61J and apparatus and associated methods for sterilization of and sterile handling of pharmaceutical materials and containers for pharmaceuticals, including bringing pharmaceuticals into form for administration to medical or veterinary patients. In one aspect, it relates to the programmed and automatic operation of such apparatus.

### Description of the Related Art

The use of controlled filling machines that dispense fluids is well known in the prior art and is becoming widespread in many industries. These machines typically include a product fluid source, a pump to propel the fluid and filling needles for directing the fluid into a container. Peristaltic pumps have a unique advantage over other pumps in that they can be cleaned by merely removing the tubing and replacing it with new tubing. The new tubing may be rapidly loaded, simplifying fluid changeover and making it contamination free. The action of peristaltic pumps is also less damaging to the fluids themselves, which may, for example, contain fragile blood cells. A major problem with peristaltic pumps, however, is precision and accuracy. Over a period of a product dispensing run, the tubing can change, resulting in a loss of precision, because the precision is directly related, among other factors, to the tube diameter and as the rotor speed.

In the pharmaceutical field, the level of tolerance required in filling containers with a pharmaceutical fluid is always very small. It is also known that, in the case of very expensive fluids, or particular or special fluids, even dangerous, toxic, poisonous or polluting ones, it is necessary to confine the filling tolerance to very low values. Depending on the type of fluid introduced, these tolerances may reach factors of 1 to 10 per thousand. With known filling systems, it is not always possible to obtain this required precision and, even when it is obtained, it is not with suitable consistency. This also leads to waste in production because of tolerances not having been met. Such waste not only causes a drop in production and an increase in costs, but also causes problems in reprocessing the containers in order to provide within them the desired quantity of fluid.

A further consideration relates to the dispensing of fluids that are dangerous, toxic, poisonous or polluting. In such cases, the reprocessing of the containers creates problems of cost, safety and contamination both of the product and of the environment. Moreover, there are fluids to be transferred that require continuous protection in order to eliminate possible contaminants, insofar as it is possible. One purpose of the present invention is therefore to perfect a method that allows the prevention of wasted production, at least in relation to expensive or dangerous, toxic, poisonous or polluting fluids, used, for example, for administration to humans, animals or plants.

The present invention relates to a rotary peristaltic pump, and, more particularly, to an apparatus and method for improving the dispensing accuracy of peristaltic pump-based filling machines. Such pumps are generally regarded as delivering a fixed volume for each fixed angular rotation of the driver. In conventional rotary peristaltic pumps the quantity of fluid delivered is generally regarded as being a fixed volume per revolution of the rotor of the pump. Rotary peristaltic pumps are preferred for many filling applications due to their ability to pump fluids through tubing without any contact between pump components and the fluid being pumped. In a typical rotary peristaltic pump system, one or more lengths of tubing are compressed by a series of rollers that rotate to squeeze the tubing against a curved wall of a stator. This provides one or more moving regions of compression along the length of tubing. Movement of the compressed region of the tubing forces fluid ahead of the moving region. In returning to its uncompressed condition, the tubing creates a partial vacuum, which results in forward flow of the fluid from the region behind the compressed region. It has been heretofore assumed that repeating cycles of the same angular rotation of a rotary peristaltic dispenser will deliver consistent quantities of product. However, a problem typically associated with such rotary peristaltic pumps is that it is difficult to obtain accurate and repeatable volume dispensing from them.

A number of approaches in dealing with this problem have been addressed in the prior art. In one approach, accuracy is sought to be enhanced by direct measurement of parameters relating to volume dispensed, motor speed and flow rate. A calculation of a calibration constant may be made to relate known increments of angular rotation of the pump to a known fluid volume. This constant may then be used to determine flow rate and total or cumulative volume dispensed based on counting angular increments of pump rotation. Encoder wheels have also been used in the prior art to improve the accuracy of rotary peristaltic pumps by monitoring the rotation of the drive shaft in small angular sectors. In the prior art examples recited hereinabove, it is assumed that the same angular distance of the driver will cause the delivery of the same volume of the product once the pump has been calibrated. When the simple calibration factor described here is utilized, a relatively large absolute error in the quantity of dispensed product results. This error is larger when peristaltic tubes of a larger inner diameter are used to achieve high production speeds. The error is even more significant when filling small volumes. The weakness of these models results from the assumption that the relation between the angle of rotation of the pump rotor and the dispensed volume of the product is a linear function with a constant coefficient linking the volume and angular distance of the driver of the rotor.

In the prior art, various approaches have been used to ensure that two consecutive dispensing cycles of a peristaltic pump would produce identical amounts of dispensed fluid. Generally, these methods function under the assumption that rotating the rotor of the pump through a selected angular displacement will produce the same volume of dispensed fluid independent of the initial angle of the rotor. However, the vagaries of the flexible tubing employed in peristaltic pumps almost inherently ensure that the volume of liquid dispensed between zero degrees rotor angle and (say) 55 degrees will not be the same as the volume of fluid dispensed when the rotor subsequently rotates from 55 degrees to 110 degrees. There are, however, examples in the prior art in which inventors have realized this fact, and have sought to re-zero the pump to the same starting angle for every dispensing cycle. The challenge in such a case is that of having to return pumped fluid to the fluid source via some manner of valved bypass fluid circuit while the pump advances to the same starting angle as employed in a previous dispensing cycle. One of the drawbacks of such an arrangement, as that the flexible tube is worn out performing no useful dispensing while the undue wear ensures that control over the dispensing is compromised.

### SUMMARY OF THE INVENTION

In a first aspect a peristaltic pump system is provided comprising: a rotary peristaltic pump comprising a stator and a rotor, the rotor driven to rotate about a rotor axis and comprising a plurality of idler rollers arranged radially equidistant about the rotor axis, each roller freely rotating about an own axis parallel to the rotor axis: a flow valve in fluid communication with an output of the pump; a fluid path in fluid communication with the valve and comprising flexible tubing disposed between the plurality of rollers and the stator such that when the rotor is rotated at least one of the plurality of rollers may exert a pressure on the tubing against the stator; a controller in communication with the pump and with the valve, the controller comprising a processor and a memory; and software instructions which when loaded in the memory and executed by the processor effect at the end of a dispensing portion of a dispensing cycle in the following order closing of the valve, operating of the pump to relieve the pressure of the rollers on the tubing, and rotating of the rotor to a start angle.

The pump may further comprise a linear actuator arranged to reciprocate the stator between a first location proximate the rotor and a second location distant from the rotor; and the software instructions when executed to relieve the pressure of the rollers on the tubing may cause the linear actuator to move the stator from the first location to the second location.

In other embodiments, the pump may further comprise a linear actuator arranged to reciprocate the rotor between a first location proximate the rotor and a second location distant from the stator; and the software instructions when executed to relieve the pressure of the rollers on the tubing may cause the linear actuator to move the rotor from the first location to the second location.

In yet other embodiments, the rollers may be retractable into the rotor; and the software instructions when executed to relieve the pressure of the rollers on the tubing may cause the rollers to be retracted.

In a further aspect a method is provided for advancing a determined amount of fluid from a fluid source through a flow valve, the method comprising: providing the fluid source, the flow valve, a rotary peristaltic pump, a controller configured to control the pump and the valve, and a fluid path placing the fluid source in fluid communication with the flow valve through the pump, the pump comprising: a stator; a rotor driven to rotate about a rotor axis, the rotor comprising a plurality of idler rollers arranged radially equidistant about the rotor axis, each roller freely rotating about an own axis parallel to the rotor axis, wherein the fluid path comprises flexible tubing disposed between the rollers and the stator; with the idler rollers exerting pressure on the flexible tubing and the valve open rotating the rotor from a start angle to a dispense angle to advance the determined amount of fluid from the fluid source through the valve; closing the valve after advancing the fluid; relaxing the pressure of the idler rollers on the tubing after closing the valve; with the pressure on the tubing relaxed and the valve closed restoring the rotor to the start angle; re-establishing the pressure of the idler rollers on the tubing; and opening the valve after re-establishing the pressure of the idler rollers on the tubing.

Providing the rotary peristaltic pump may comprise providing the peristaltic pump with the stator arranged to reciprocate between a first location proximate the rotor and a second location distant from the rotor; and relaxing the pressure of the idler rollers on the tubing may comprise moving the stator from the first location to the second location.

In another embodiment of the method, providing the rotary peristaltic pump may comprise providing the peristaltic pump with the rotor arranged to reciprocate between a first location proximate the stator and a second location distant from the stator; and relaxing the pressure of the idler rollers on the tubing may comprise moving the rotor from the first location to the second location.

In yet a further embodiment of the method, providing the rotary peristaltic pump may comprise providing the peristaltic pump with the idler rollers arranged to be retractable into the rotor; and relaxing the pressure of the idler rollers on the tubing may comprise retracting the rollers into the rotor.

In a further aspect, as method is provided for aseptically filling a container with a pharmaceutical fluid, the method comprising: providing a fluid source, a fill needle and container disposed within a sterile isolator, a flow valve, a rotary peristaltic pump, a controller configured to control the pump and the valve, and a fluid path extending between the fluid source and the flow valve and from the valve to the fill needle disposed above an opening of a first container, the pump comprising: a stator; a rotor driven to rotate about a rotor axis, the rotor comprising a plurality of idler rollers arranged radially equidistant about the rotor axis, each roller freely rotating about an own axis parallel to the rotor axis, wherein the fluid path comprises flexible tubing disposed between the rollers and the stator; moving at least one of the container and the fill needle to locate the fill needle over an opening in the container: with the idler rollers exerting pressure on the flexible tubing and the valve open rotating the rotor from a start angle to a dispense angle to advance the determined amount of fluid from the fluid source through the valve and the fill needle into the container; closing the valve after advancing the fluid; relaxing the pressure of the idler rollers on the tubing after closing the valve; with the pressure on the tubing relaxed and the valve closed restoring the rotor to the start angle; re-establishing the pressure of the idler rollers on the tubing; and opening the valve after re-establishing the pressure of the idler rollers on the tubing.

Providing the rotary peristaltic pump may comprise providing the peristaltic pump with the stator arranged to reciprocate between a first location proximate the rotor and a second location distant from the rotor; and relaxing the pressure of the idler rollers on the tubing may comprise moving the stator from the first location to the second location.

In another embodiment of the method, providing the rotary peristaltic pump may comprise providing the peristaltic pump with the rotor arranged to reciprocate between a first location proximate the stator and a second location distant from the stator; and relaxing the pressure of the idler rollers on the tubing may comprise moving the rotor from the first location to the second location.

In yet a further embodiment of the method, providing the rotary peristaltic pump may comprise providing the peristaltic pump with the idler rollers arranged to be retractable into the rotor; and relaxing the pressure of the idler rollers on the tubing may comprise retracting the rollers into the rotor.

In yet a further aspect, a method is provided for aseptically filling a plurality of containers with a pharmaceutical fluid, the method comprising: (a) providing a fluid source, disposed within a sterile isolator a fill needle and the plurality of containers within a container nest, a flow valve, a rotary peristaltic pump, a controller configured to control the pump and the valve, and a fluid path extending between the fluid source and the flow valve and from the valve to the fill needle disposed above an opening of a first container, the pump comprising: a stator; a rotor driven to rotate about a rotor axis, the rotor comprising a plurality of idler rollers arranged radially equidistant about the rotor axis, each roller freely rotating about an own axis parallel to the rotor axis, wherein the fluid path comprises flexible tubing disposed between the rollers and the stator; (b) moving at least one of the container nest and the fill needle to locate the fill needle over an opening of a first of the plurality of containers: (c) with the idler rollers exerting pressure on the flexible tubing and the valve open rotating the rotor from a start angle to a dispense angle to advance the determined amount of fluid from the fluid source through the valve and the fill needle into the first of the plurality of containers; (d) closing the valve after advancing the fluid: (e) relaxing the pressure of the idler rollers on the tubing after closing the valve: (f) with the pressure on the tubing relaxed and the valve closed restoring the rotor to the start angle; (g) re-establishing the pressure of the idler rollers on the tubing: (h) moving at least one of the container nest and the fill needle to locate the fill needle over an opening of another of the plurality of containers; and (i) opening the valve; (j) repeating steps (c) to (i) until the plurality of containers have been filled with fluid.

Providing the rotary peristaltic pump may comprise providing the peristaltic pump with the stator arranged to reciprocate between a first location proximate the rotor and a second location distant from the rotor; and relaxing the pressure of the idler rollers on the tubing may comprise moving the stator from the first location to the second location.

In an alternative embodiment of the method, providing the rotary peristaltic pump may comprise providing the peristaltic pump with the rotor arranged to reciprocate between a first location proximate the stator and a second location distant from the stator; and relaxing the pressure of the idler rollers on the tubing may comprise moving the rotor from the first location to the second location.

In yet a further embodiment of the method, providing the rotary peristaltic pump may comprise providing the peristaltic pump with the idler rollers arranged to be retractable into the rotor; and relaxing the pressure of the idler rollers on the tubing may comprise retracting the rollers into the rotor.

### BRIEF DESCRIPTION OF THE DRAWINGS

The features and objects of this invention, and the manner of attaining them, will become more apparent and the invention itself will be better understood by reference to the following description of an embodiment of the invention taken in conjunction with the accompanying drawings, wherein:
**FIG. 1** is a drawing of a first embodiment of a peristaltic pump-based apparatus for filling pharmaceutical containers with a pharmaceutical fluid product.
**FIG. 2A** is close-up view of the peristaltic pump of **FIG. 1** with the stator of the pump in a first location.
**FIG. 2B** is close-up view of the peristaltic pump of **FIG. 1** with the stator of the pump in a second location.
**FIG. 3A** to **FIG. 3G** shows a series of steps in operating the peristaltic pump-based apparatus of **FIG. 1****.**
**FIG. 4** is a flow diagram of a method for advancing a determined amount of fluid from a fluid source through a flow valve by means of a peristaltic pump having a reciprocating stator
**FIG. 5A** to **FIG. 5G** shows a series of steps in operating a peristaltic pump-based apparatus of FIG.1 using an alternative embodiment of the pump
**FIG. 6** is a flow diagram of a method of advancing a determined amount of fluid from a fluid source through a flow valve by means of a peristaltic pump having a reciprocating rotor.
**FIG. 7** is a view of an alternative embodiment of the peristaltic pump of **FIG. 1** wherein the pump has retractable idler rollers. For purposes of clarity, the stator of the pump is not shown and only two idler rollers are shown.
**FIG. 8A** to **FIG. 8G** shows a series of steps in operating the peristaltic pump-based apparatus of **FIG. 1** employing the pump of **FIG. 7****.**
**FIG. 9** is a flow diagram of a method for advancing a determined amount of fluid from a fluid source through a flow valve by means of a peristaltic pump having retractable rotors.

Corresponding reference characters indicate corresponding parts throughout the several views. Although the drawings represent embodiments of the present invention, the drawings are not necessarily to scale and certain features may be exaggerated in order to better illustrate and explain the present invention. The flow charts are also representative in nature, and actual embodiments of the invention may include further features or steps not shown in the drawings. The exemplification set out herein illustrates an embodiment of the invention, in one form, and such exemplifications are not to be construed as limiting the scope of the invention in any manner.

### DESCRIPTION OF EMBODIMENTS OF THE PRESENT INVENTION

The embodiments disclosed below are not intended to be exhaustive or limit the invention to the precise form disclosed in the following detailed description. Rather, the embodiments are chosen and described so that others skilled in the art may utilize their teachings.

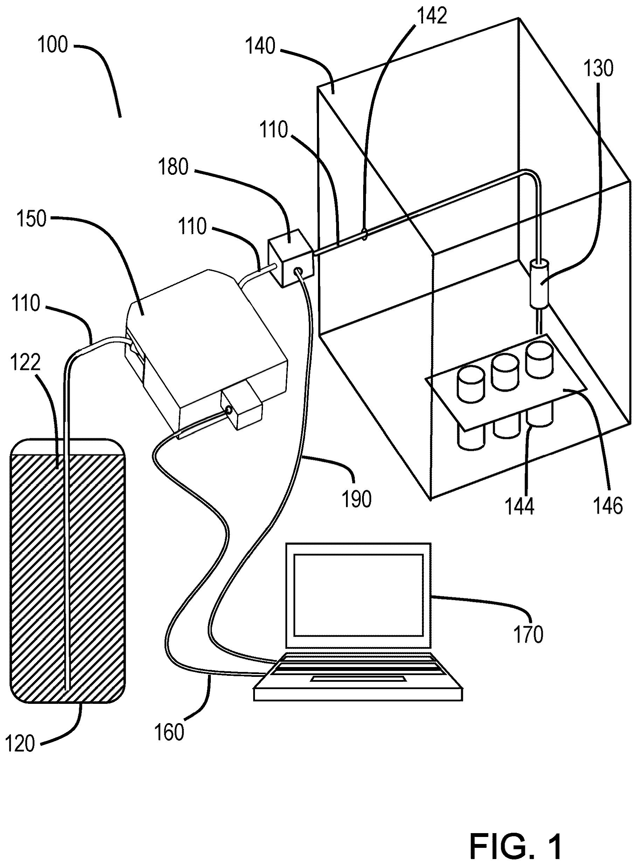

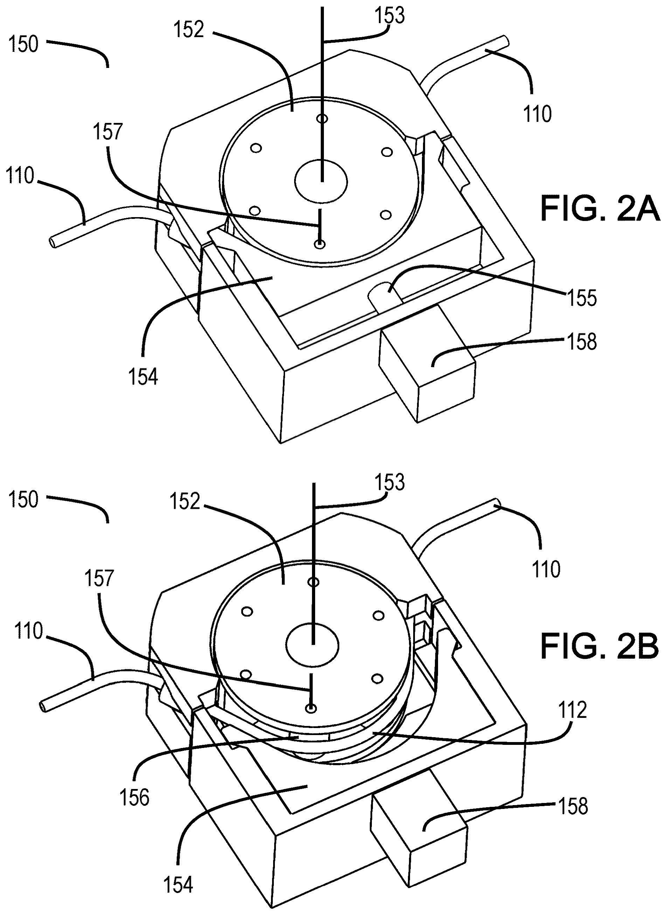

In **FIG. 1****,** apparatus **100** is shown for aseptically filling container **144** within sterile isolator **140** with pharmaceutical fluid **122** from fluid source container **120** via fluid path **110,** peristaltic pump **150** with a precipitating stator, shut-off valve **180,** and fill needle **130.** Peristaltic pump **150** is controlled by controller **170** via pump control line **160** and shut-off valve **180** is controlled by controller **170** via valve control line **190.** Fluid path **110** enters sterile isolator **140** via port **142,** which may be hermetically sealed in order to retain the sterile state of the interior of isolator **140.** At least within pump **150,** the fluid path is defined by flexible tube **112,** as shown in **FIG. 2B**

**FIG. 2A** and **FIG. 2B** show peristaltic pump **150** of **FIG.1** in more detail and in two different states. Pump **150** comprises rotor **152** driven about rotor axis **153,** rotor **152** bearing a plurality of idler rollers **156** arranged radially equidistant about rotor axis **153** and free to rotate about axes **157** parallel to rotor axis **153.** Reciprocating stator **154** reciprocates along the direction of shaft **155** under the action of actuator **158.** Actuator **158** is under the control of controller **170** of **FIG. 1****.** Stator **154** is arranged to reciprocate between a first location proximate rotor **152,** as shown in **FIG. 2A****,** and a second location distant from rotor **152,** as shown in **FIG. 2B****,** flexible tubing **110** being disposed between at least one of the plurality of idler rollers **156** and stator **154,** as may also be seen in **FIG. 2B****.** When stator **154** is in the first location, idler rollers **156** exert pressure on flexible tubing **110** and pump **150** functions as a classic peristaltic pump, rollers **156** advancing fluid **122** along flexible tube **112** as they move and rotate around their own axes **157** during rotation of rotor **152.** In **FIG. 1****,** and in **FIG. 2A** and **FIG. 2B****,** the rotation of rotor **152** is anti-clockwise when advancing fluid **122** from left to right through tube **112** in the diagrams. When stator **154** is in the second location, rollers **156** do not exert pressure on flexible tubing **112** and rotor **152** may be rotated without advancing fluid **122** through tube **112.**

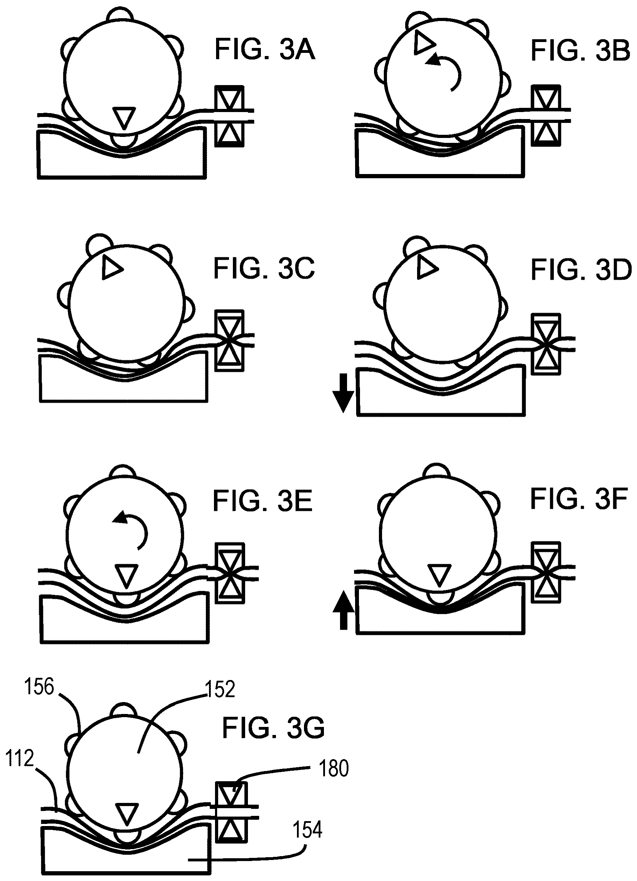

**FIG. 3A-G** schematically show a series of states of valve **180** of **FIG. 1** and of peristaltic pump **150** of **FIG. 2A** and **FIG. 2B** as part of the operating of system **100** of **FIG.1****.** In the interest of clarity, only **FIG. 3G** is labeled, thereby avoiding obfuscation of the series of schematics. Purely for the purposes of explanation of the operation, rotor **152** is labeled with a triangle in this series of schematics, the triangle serving as reference to show the angle of rotation of rotor **152.** **FIG. 3A** shows pump **150** with stator **154** in the first location proximate the rotor and compressing flexible tube **112** against one of rotors **156.** Rotor **152** is shown as rotating anti-clockwise to advance fluid to the right and through valve **180,** valve **180** being open to allow through the fluid. Within **FIG. 1****,** the fluid is being advanced to dispensing needle **130** when pump **150** and valve **180** are in the state shown in **FIG. 3A****.**

**FIG. 3B** shows rotor **152** as having rotated through a dispensing angle required for a single dispensing cycle. For the sake of explaining the working of this system, the dispensing angle is taken to be approximately 210 degrees, or multiple of 360 degrees plus 210 degrees. The angle selected in a practical dispensing cycle would be based on the amount of fluid required to be dispensed. At this point in the cycle, valve **180** is still open. **FIG. 3C** represents the next step in the dispensing cycle in which valve **180** is closed. **FIG. 3A-C**together therefore represent the dispensing portion of the dispensing cycle.

In **FIG. 3D****,** stator **154** is moved to the second location distant from rotor **152,** thereby relieving the pressure on tube **112.** **FIG. 3E** shows rotor **152** rotated to return it to its starting angle while valve **180** is still closed and stator **154** is still in the second location. Since rollers **156** are not exerting pressure on tube **112** during this rotation, no fluid is being advanced. In some embodiments, the relative movement of the rotor and stator involves physically removing the plurality of rollers from contacting the tubing. There is also no wear on the tube **112.** In **FIG. 3F****,** stator **154** is moved back to the first location, thereby restoring the pressure on tube **112,** while **FIG. 3G** shows valve **180** opened, so that **FIG. 3G** restores the system to exactly the same state as in **FIG. 3A****,** ready to initiate a next dispensing cycle. **Figures 3D-G** therefore show the reset portion of the dispensing cycle, one complete dispensing cycle comprising a dispensing portion and a reset portion.

All of the above steps may be executed automatically by controller **170** operating actuator **158** via pump control line **160** and valve **180** via valve control line **190,** and alternatively controller **170** may wirelessly control actuator **158** and valve **180** through a telecommunications protocol, e.g. Bluetooth or Wi-Fi. To this end, controller **170** may comprise among its hardware a processor and a memory. A set of instructions may be loaded into the memory. The instructions, when executed by the processor, may perform the steps of: moving stator **154** to the first location, opening valve **180,** and then rotating rotor **152** from a start angle to a dispense angle to allow a determined amount of fluid to flow from fluid source **120** through fluid path **110** and through valve **180;** and, subsequent to rotating rotor **152** to the dispense angle, closing valve **180,** relaxing pressure on tubing **112** by moving stator **154** to the second location, and then restoring rotor **152** to the start angle without causing fluid to flow through valve **180.** When a plurality of dispenses into a plurality of containers **144** need to be made, controller **170** may repeat the cycle described above for each of containers **144** in the plurality of containers.

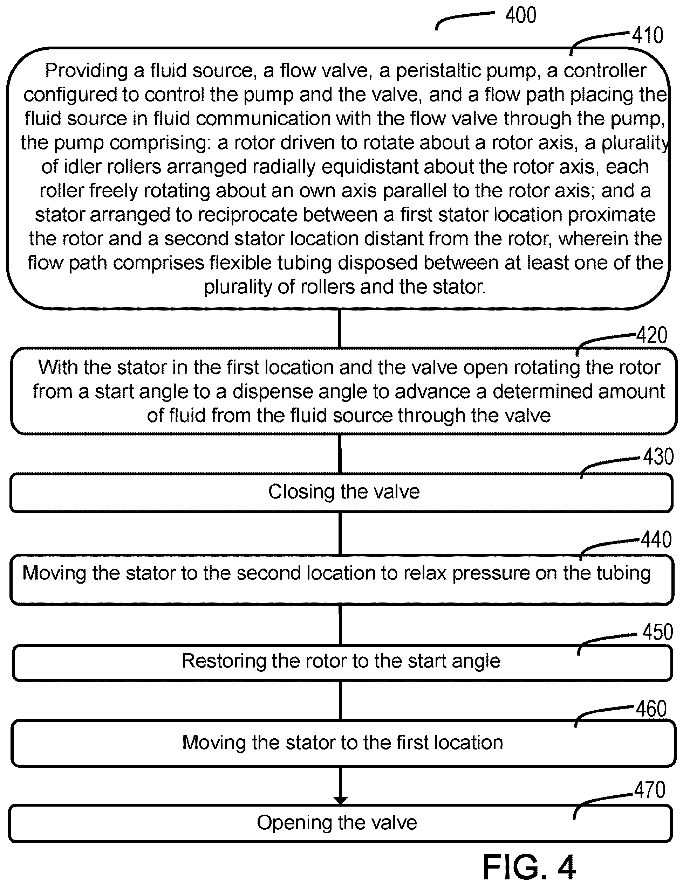

In another aspect, described at the hand of the flow chart of **FIG. 4** and the apparatus of **FIG. 1****,** **FIG. 2A** and **FIG. 2B****,** as well as **FIG. 3A-G,**there is presented method [400] for aseptically filling first container **144** with pharmaceutical fluid **122,** the method comprising: providing **[410]** fluid source **120,** flow valve **180,** rotary peristaltic pump **150,** controller **170** configured to control pump **150** and valve **180,** and fluid path **110** extending between fluid source **120** and flow valve **180** and from valve **180** to fill needle **130** disposed above an opening of first container **144,** pump **150** comprising: rotor **152** driven to rotate about rotor axis **153,** a plurality of idler rollers **156** arranged radially equidistant about rotor axis **153,** each roller **156** freely rotating about an own axis **157** parallel to rotor axis **153;** and stator **154** arranged to reciprocate between a first location proximate rotor **152** and a second location distant from rotor **152,** wherein fluid path **110** comprises flexible tubing **112** disposed between at least one of the plurality of rollers **156** and stator **154;** with stator **154** in the first location and valve **180** open rotating **[420]** rotor **152** from a start angle to a dispense angle to advance a determined amount of fluid **122** from fluid source **120** through fluid path **110** and through valve **180** to fill needle **130;** closing **[430]** valve **180;** moving **[440]** stator **154** to the second location to relax pressure on tubing **112;** restoring **[450]** rotor **152** to the start angle; moving **[460]** stator **154** to the first location; and opening **[470]** valve **180.**

Method **[400]** may further comprise providing first container **144** as one of a plurality of containers held in container nest **146.** The method may yet further comprise, after rotating **[420]** rotor **152** from a start angle to a dispense angle and closing **[430]** valve **180,** one of moving an opening of a second of the plurality of containers under fill needle **130** and moving fill needle **130** to be above an opening of a second of the plurality of containers. Moving an opening of the second of the plurality of containers may comprise moving container nest **146.** The method may further comprise repeating steps **[420]** to **[470]** to advance again the determined amount of fluid **122** from fluid source **120** along fluid path **110** through tubing **112** and through valve **180** to fill needle **130** and from there into the second of the plurality of containers.

In another implementation, the pressure on tube **112** is relieved not by moving stator **154,** but to instead reciprocate rotor **152** between a first location proximate stator **154** and a second location distant from stator **154.** In this implementation, stator **154** is kept stationary and the rest of peristaltic pump **150** is allowed to move with respect to stator **154** under the action of actuator **158.**

In this implementation, **FIG. 2A** and **FIG. 2B** may be viewed as showing peristaltic pump **150** of **FIG. 1** in more detail and in two different states. Pump **150** comprises rotor **152** driven about rotor axis **153,** rotor **152** bearing a plurality of idler rollers **156** arranged radially equidistant about rotor axis **153** and free to rotate about axes **157** parallel to rotor axis **153.** Reciprocating rotor **152** reciprocates along the direction of shaft **155** under the action of actuator **158.** Actuator **158** is under the control of controller **170** of **FIG 1****.** Rotor **152** is arranged to reciprocate between a first location proximate stator **154,** as shown in **FIG. 2A****,** and a second location distant from stator **154,** as shown in **FIG. 2B****,** flexible tubing **112** being disposed between at least one of the plurality of idler rollers **156** and stator **154,** as may also be seen in **FIG. 2B****.** When rotor **152** is in the first location, idler rollers **156** exert pressure on flexible tubing **112** and pump **150** functions as a classic peristaltic pump, rollers advancing fluid **122** along tube **112** as they move and rotate around their own axes **157** during rotation of rotor **152.** In **FIG. 1** and in **FIG. 2A** and **FIG. 2B****,** the rotation of rotor **152** is anti-clockwise when advancing fluid **122** from left to right through tube **112** in the diagrams. When rotor **152** is in the second location, rollers **156** do not exert pressure on flexible tubing **112** and rotor **152** may be rotated without advancing fluid **122** through tube **112.**

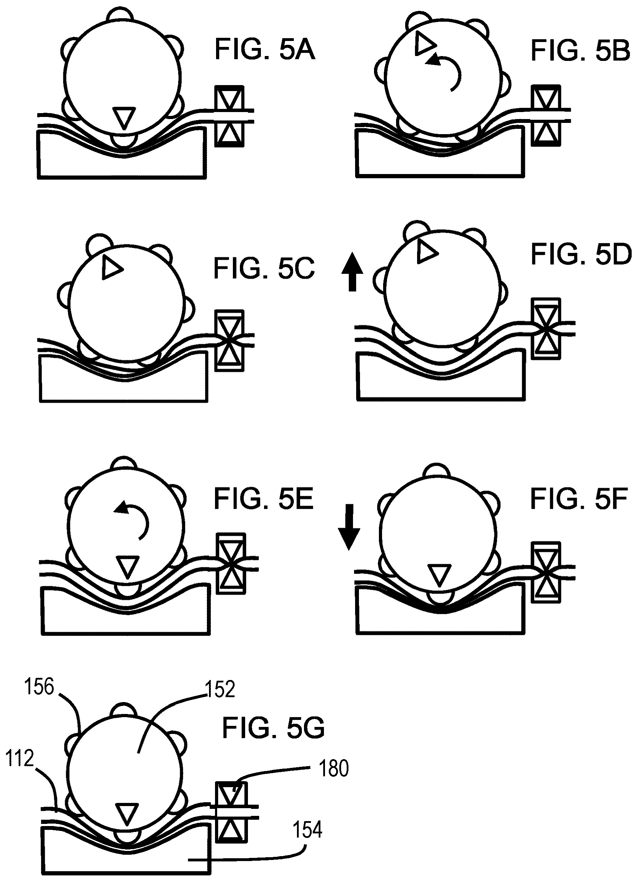

**Figures 5A-G**schematically show a series of states of valve **180** of **FIG. 1** and of peristaltic pump **150** of **FIG. 2A** and **FIG. 2B** as part of the operating of system **100** of **FIG. 1****.** In the interest of clarity, only **FIG. 5G** is labeled, thereby avoiding obfuscation of the series of schematics. Purely for the purposes of explanation of the operation, rotor **152** is labeled with a triangle in this series of schematics, the triangle serving as reference to show the angle of rotation of rotor **152.** **FIG. 5A** shows pump **150** with rotor **152** in the first location proximate stator **154** and at least one of idler rollers **156** compressing flexible tube **112** against rotor **152.** Rotor **152** is shown as rotating anti-clockwise to advance fluid to the right and through valve **180,** valve **180** being open to allow through the fluid. Within **FIG. 1****,** the fluid is being advanced to dispensing needle **130** when pump **150** and valve **180** are in the state shown in **FIG. 5A****.**

**FIG. 5B** shows rotor **152** as having rotated through a dispensing angle required for a single dispensing cycle. For the sake of explaining the working of this system, the dispensing angle is taken to be approximately 210 degrees, or multiple of 360 degrees plus 210 degrees. The angle selected in a practical dispensing cycle would be based on the amount of fluid required to be dispensed. At this point in the cycle, valve **180** is still open. **FIG. 5C** represents the next step in the dispensing cycle in which valve **180** is closed. **FIG. 5A-C**together therefore represent the dispensing portion of the dispensing cycle.

In **FIG. 5D****,** rotor **152** is moved to the second location distant from stator **154,** thereby relieving the pressure on tube **112.** **FIG. 5E** shows rotor **152** rotated to return it to its starting angle while valve **180** is still closed and rotor **152** is still in the second location. Since rollers **156** are not exerting pressure on tube **112** during this rotation, no fluid is being advanced, nor is any fluid being discharged or otherwise diverted. There is also no wear on tube **112.** In **FIG. 5F****,** rotor **152** is moved back to the first location, thereby restoring the pressure on tube **112,** while **FIG. 5G** shows valve **180** opened, so that **FIG. 5G** restores the system to exactly the same state as in **FIG. 5A****,** ready to initiate a next dispensing cycle. **Figures 5D-G** therefore show the reset portion of the dispensing cycle, one complete dispensing cycle comprising a dispensing portion and a reset portion.

All of the above steps may be executed automatically by controller **170** operating actuator **158** via pump control line **160** and valve **180** via valve control line **190.** To this end, controller **170** may comprise among its hardware a processor and a memory, and alternatively controller **170** may wirelessly control actuator **158** and valve **180** through a telecommunications protocol, e.g. Bluetooth or Wi-Fi. A set of instructions may be loaded into the memory. The instructions, when executed by the processor, may perform the steps of: moving rotor **152** to the first location, opening valve **180,** and then rotating rotor **152** from a start angle to a dispense angle to allow a determined amount of fluid to flow from fluid source **120** along flow path **110** through tubing **112** and through valve **180;** and, subsequent to rotating rotor **152** to the dispense angle, closing valve **180,** relaxing pressure on tubing **112** by moving rotor **152** to the second location, and then restoring rotor **152** to the start angle without causing fluid to flow through valve **180.** When a plurality of dispensings into a plurality of containers **144** need to be made, controller **170** may repeat the cycle described above for each of containers **144** in the plurality of containers.

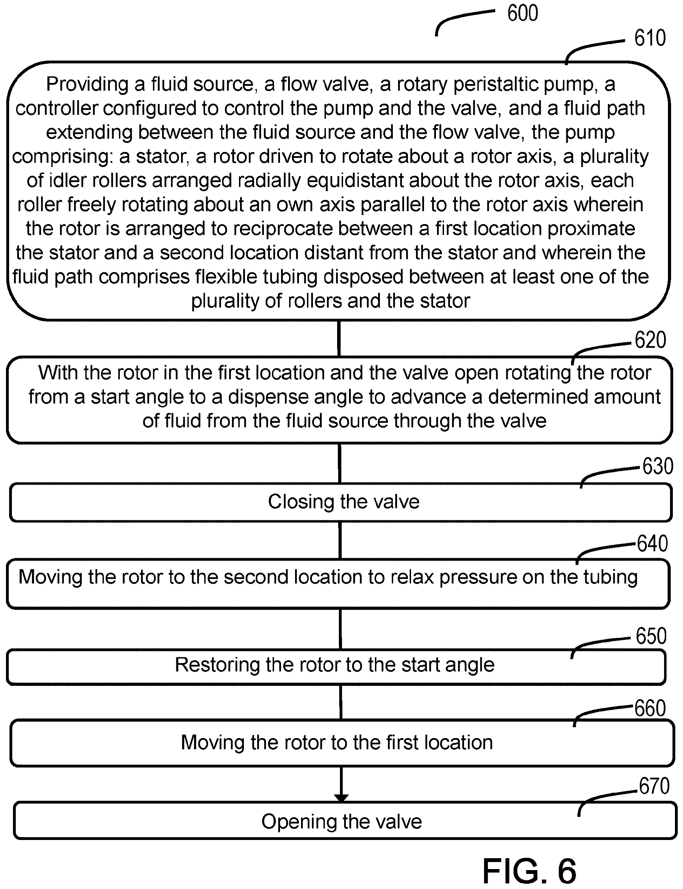

In another aspect, described at the hand of the flow chart of **FIG. 6** and the apparatus of **FIG. 1****,** **FIG. 2A** and **FIG. 2B**, as well as **FIG. 5A-G,**there is presented method **[600]** for aseptically filling first container **144** with pharmaceutical fluid **122,** the method comprising: providing **[610]** fluid source **120,** flow valve **180,** rotary peristaltic pump **150,** controller **170** configured to control pump **150** and valve **180,** and fluid path **110** extending between fluid source **120** and flow valve **180** and from valve **180** to fill needle **130** disposed above an opening of first container **144,** pump **150** comprising: stator **154,** rotor **152** driven to rotate about rotor axis **153,** a plurality of idler rollers **156** arranged radially equidistant about rotor axis **153,** each roller **156** freely rotating about an own axis **157** parallel to rotor axis **153** wherein rotor **152** is arranged to reciprocate between a first location proximate stator **154** and a second location distant from stator **154,** wherein fluid path **110** comprises flexible tubing **112** disposed between at least one of the plurality of rollers **156** and stator **154;** with rotor **152** in the first location and valve **180** open rotating **[620]** rotor **152** from a start angle to a dispense angle to advance a determined amount of fluid **122** from fluid source **120** along fluid path **110** through tubing **112** and through valve **180** to fill needle **130;** closing **[630]** valve **180;** moving [640] rotor **152** to the second location to relax pressure on tubing **112;** restoring **[650]** rotor **152** to the start angle; moving **[660]** rotor **152** to the first location; and opening **[670]** valve **180.**

Method **[600]** may further comprise providing first container **144** as one of a plurality of containers held in container nest **146.** The method may yet further comprise, after rotating **[620]** rotor **152** from a start angle to a dispense angle and closing **[630]** valve **180,** one of moving an opening of a second of the plurality of containers under fill needle **130** and moving fill needle **130** to be above an opening of a second of the plurality of containers. Moving an opening of the second of the plurality of containers may comprise moving container nest **146.** The method may further comprise repeating steps **[620]** to **[670]** to advance again the determined amount of fluid **122** along fluid path **110** from fluid source **120** through tubing **112** and through valve **180** to fill needle **130** and from there into the second of the plurality of containers.

In an embodiment of a second implementation of system **100** of the present invention shown in **FIG. 1****,** further mechanisms for reciprocating the rotor between the first and the second rotor locations are contemplated, the further mechanisms comprising moving only the rotor and no other portions of the pump, or moving as little mass as possible in addition to the rotor.

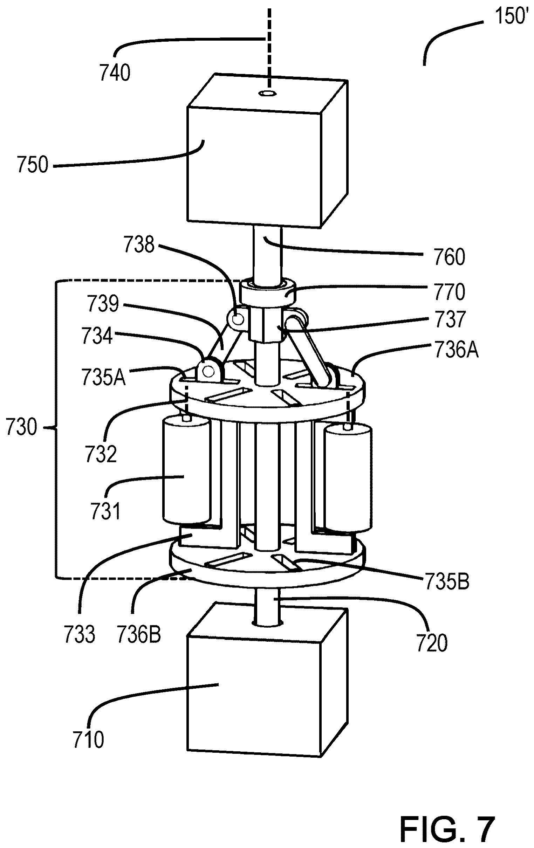

In yet a further implementation of the system of **FIG. 1****,** the idler rollers of the rotor do not rotate about fixed axes arranged about the rotor axis. Instead, as shown in **FIG. 7****,** rollers **731** are retractable into rotor **730** to a degree that relieves the pressure on tube **112** bearing fluid **122.** In such an implementation, there is no requirement for either the rotor or the stator to reciprocate during operation of the pump, as rollers **731** may be retracted during the reset portion of the dispensing cycle.

In **FIG. 7****,** rotary peristaltic pump **150'** is shown with its stator and tubing omitted for the sake of clarity. Pump **150'** may be employed in the same arrangement as in **FIG. 1****,** in which arrangement it replaces pump **150.** Pump engine **710** drives pump axle **720** on which is mounted rotor **730,** thereby driving rotor **730** to rotate about pump axis **740.** Axis **740** also serves as rotor axis. Linear actuator **750** is arranged to extend and retract actuator rod **760** along axis **740.** Actuator rod **760** is fixed to the inner ring of ball bearing **770,** allowing thereby the outer ring of ball bearing **770,** along with all systems attached to that outer ring, to rotate freely about actuator rod **760.** Bushing **737** comprising six linkage mounts **738** is mounted fixedly to the outer ring of ball bearing **760.** For the sake of clarity, only two linkage mounts **738** are shown in **FIG. 1** of which only one is labeled. Rotor **730** further comprises first and second rotor assembly plates **736A** and **736B** which each engage with six idler roller subassemblies to be described below. For the sake of clarity only two of the six idler roller subassemblies are shown in **FIG. 1****,** and only one of the two has numbered elements. In, general rotor **730** may comprise any number of idler roller subassemblies.

Idler rollers **731** are held in roller mounts **733** which allow rollers **731** to rotate freely about roller axes **732.** Each of roller mounts **733** has two linkage mounts **734,** one for sliding within slide guide **735A** in rotor assembly plate **736A,** and another obscured by rotor assembly plate **736B** and arranged for sliding in a slide guide (obscured in **FIG. 1****)** within rotor assembly plate **736B.** With four of the idler roller assemblies omitted from **FIG. 1****,** four of slide guides **735B** in rotor assembly plate **736B** are visible. Linkage mount **734** is connected to linkage mount **738** on bushing **737** by linkage **739.** Linkage **739** is free to rotate with linkage mount **734** and within linkage mount **738.**

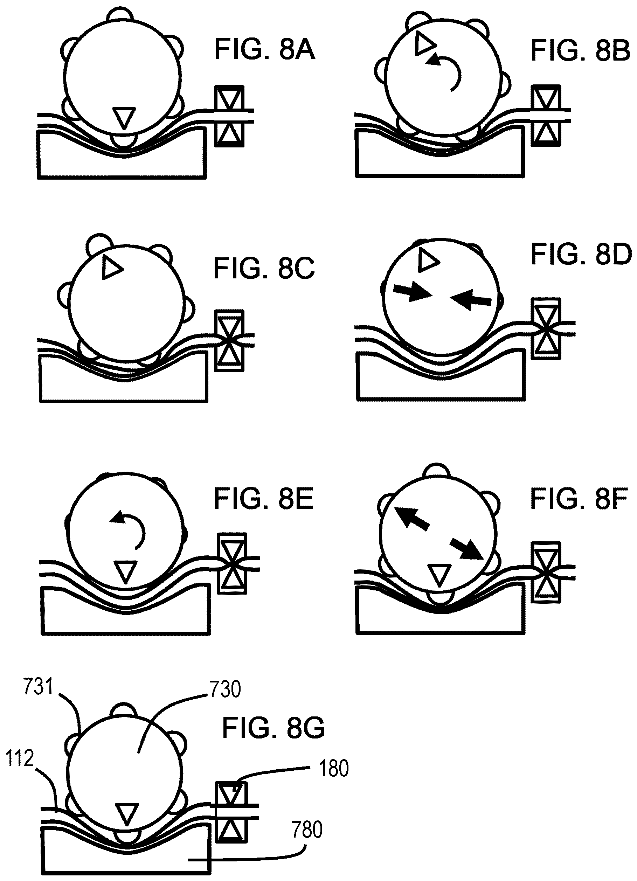

In operation, linear actuator **750** may extend actuator rod **760** along pump axis **740** and thereby causes bushing **737** to move closer to rotor assembly plate **736A.** This causes linkage **739** to rotate with both linkage mounts **734** and **738** and to exert a lateral force on rotor mount **733,** which in turn causes linkage mounts **734** to slide outward within their respective slide guides. This action positions idler rollers **731** further from pump axis **740.** With reference to **FIG. 8****,** when extended in this fashion, rollers **731** may exert pressure on flexible tubing **112** in pump **150'** by pressing flexible tubing **112** against suitable stator **780.** When linear actuator **750** retracts rod **760,** that pressure is relieved.

**Figures 8A-G**schematically show a series of states of valve **180** of **FIG. 1** and of peristaltic pump **150'** of **FIG. 7** as part of the operating of system **100** of **FIG. 1****.** In the interest of clarity, only **FIG. 8G** is labeled, thereby avoiding obfuscation of the series of schematics. Purely for the purposes of explanation of the operation, rotor **730** is labeled with a triangle in this series of schematics, the triangle serving as reference to show the angle of rotation of rotor **730.** **FIG. 8A** shows pump **150'** with at least one of idler rollers **731** compressing flexible tube **112** against stator **780.** Rotor **730** is shown as rotating anti-clockwise to advance fluid to the right and through valve **180,** valve **180** being open to allow through the fluid. Within **FIG. 1****,** the fluid is being advanced to dispensing needle **130** when pump **150'** and valve **180** are in the state shown in **FIG. 8A****.**

**FIG. 8B** shows rotor **152** as having rotated through a dispensing angle required for a single dispensing cycle. For the sake of explaining the working of this system, the dispensing angle is taken to be approximately 210 degrees, or multiple of 360 degrees plus 210 degrees. The angle selected in a practical dispensing cycle would be based on the amount of fluid required to be dispensed. At this point in the cycle, valve **180** is still open. **FIG. 8C** represents the next step in the dispensing cycle in which valve **180** is closed. **FIG. 8A-C** together therefore represent the dispensing portion of the dispensing cycle.

In **FIG. 8D****,** linear actuator **750** retracts actuator rod **760** to retract idler rollers **731** closer to pump axis **740,** relieving thereby the pressure on tube **112.** **FIG. 8E** shows rotor **152** rotated to return it to its starting angle while valve **180** is still closed and rotor **152** is still in the second location. Since rollers **731** are not exerting pressure on tube **112** during this rotation, no fluid is being advanced. There is also no wear on tube **112.** In **FIG. 8F****,** linear actuator **750** extends rod **760** back to its prior position, thereby restoring rollers **731** to their prior positions relative to axis **740,** thereby re-establishing the pressure of rollers **731** on tube **110.** **FIG. 8G** shows valve **180** opened, thereby restoring system to exactly the same state as in **FIG. 8A****,** ready to initiate a next dispensing cycle. **Figures 8D-G**therefore show the reset portion of the dispensing cycle, one complete dispensing cycle comprising a dispensing portion and a reset portion.

All of the above steps may be executed automatically by controller **170** operating actuator **750** via pump control line **160** and valve **180** via valve control line **190.** To this end, controller **170** may comprise among its hardware a processor and a memory, and alternatively controller **170** may wirelessly control actuator **158** and valve **180** through a telecommunications protocol, e.g. Bluetooth or Wi-Fi. A set of instructions may be loaded into the memory. The instructions, when executed by the processor, may perform the steps of: extending rollers **731** to a first radial distance from pump axis **740,** opening valve **180,** and then rotating rotor **730** from a start angle to a dispense angle to allow a determined amount of fluid to flow from fluid source **120** along fluid path **110** through tubing **112** and through valve **180:** and, subsequent to rotating rotor **730** to the dispense angle, closing valve **180,** relaxing pressure on tubing **112** by retracting rollers **731** to a second radial distance closer to pump axis **740,** and then restoring rotor **730** to the start angle without causing fluid to flow through valve **180.** When a plurality of dispensings into a plurality of containers **144** need to be made, controller **170** may repeat the cycle described above for each of the containers **144** in the plurality of containers.

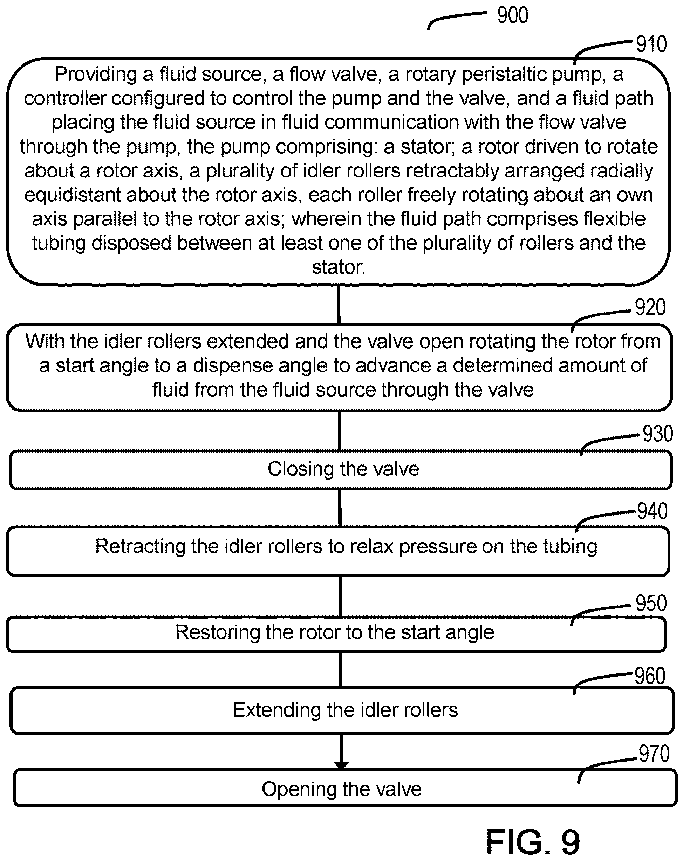

With reference to **FIG. 1****,** **FIG. 7** and **Figures 8A-E,**along with the flow chart in **FIG. 9****,** there is presented method **[900]** for aseptically filling first container **144** with pharmaceutical fluid **122,** the method comprising: providing **[910]** fluid source **120,** flow valve **180,** rotary peristaltic pump **150',** controller **170** configured to control pump **150'** and valve **180,** and fluid path **110** placing fluid source **120** in fluid communication with flow valve **180** through pump **150',** pump **150'** comprising: a stator **780;** a rotor **730** driven to rotate about rotor axis **740,** a plurality of idler rollers **731** retractably arranged radially equidistant about rotor axis **740,** each roller freely rotating about an own roller axis **732** parallel to rotor axis **740** wherein fluid path **110** comprises flexible tubing **112** disposed between at least one of the plurality of rollers **731** and stator **780:** with idler rollers **731** extended and valve **180** open rotating **[920]** rotor **730** from a start angle to a dispense angle to advance a determined amount of fluid **122** from fluid source **120** through valve **180** and to dispensing needle **130;** closing **[930]** valve **180;** retracting **[940]** idler rollers **731** to relax pressure on tubing **112**; restoring **[950]** rotor **730** to the start angle; extending **[960]** idler rollers **731** to establish pressure on tubing **112;** and opening **[970]** valve **180.**

Method **[900]** may further comprise providing first container **144** as one of a plurality of containers held in container nest **146.** The method may yet further comprise, after rotating **[920]** rotor **730** from a start angle to a dispense angle and closing **[930]** valve **180,** one of moving an opening of a further one of the plurality of containers under fill needle **130** and moving fill needle **130** to be above an opening of a second of the plurality of containers. The moving the opening of the further one of the plurality of containers may comprise moving container nest **146.** The method may further comprise repeating steps **[920]** to **[970]** to advance the determined amount of fluid **122** along fluid path **110** from fluid source **120** through tubing **112** and through valve **180** to fill needle **130** and from there into further ones of the plurality of containers **144** until the plurality of containers **144** have been filled with fluid.

In any of the embodiments of the present invention, the only portion of the fluid path that is required to be a flexible tube is the portion acted upon by the idler rollers. The fluid path from fluid source **120** to pump **150, 150',** and/or the fluid path from pump **150, 150'** to flow valve **180,** and/or the fluid path from flow valve **180** to fill needle **130** may be, for example, rigid medical grade stainless steel or made of a another material that meets pharmaceutical specifications. Flow valve **180** may also be mounted directly on the output of pump **150, 150'.** In any of the embodiments of the present invention, the moving of container nest **146** may be by means of a conveyor belt; a robotic arm as described in US Patent Application Publication US 2009/0223592 A1 and in PCT Application Publication Number WO 2013/016248 A1**,** both wholly incorporated herein by reference; by means of a rotary stage as described in US Patent Application Publication US 2018/0072446 A1**,** wholly incorporated herein by reference; or by any precision means compatible with the environmental requirements of chamber **140.** In other embodiments, fill needle **130** may be moved by suitable means, for example without limitation, a robotic arm, including, without limitation, an articulated robotic arm.

Each of the three embodiments of the method for advancing a determined amount of fluid **122** from fluid source **120** through flow valve **180** comprises: providing fluid source **120,** flow valve **180,** rotary peristaltic pump **150, 150',** controller **170** configured to control pump **150, 150'** and valve **180,** and fluid path **110** placing fluid source **120** in fluid communication with flow valve **180** through pump **150, 150',** pump **150, 150'** comprising: stator **154, 780;** rotor **152, 730** driven to rotate about rotor axis **153, 740,** rotor **152, 730** comprising a plurality of idler rollers **156, 731** arranged radially equidistant about rotor axis **153, 740,** each roller **156, 731** freely rotating about an own roller axis **157, 732** parallel to rotor axis **153,740** wherein fluid path **110** comprises flexible tubing **112** disposed between rollers **156, 731** and stator **154, 780;** rotating rotor **152, 730** from a start angle to a dispense angle with idler rollers **156, 731** exerting pressure on flexible tubing **112** and valve **180** open to advance the determined amount of fluid from fluid source **120** through valve **180** and to dispensing needle **130:** closing valve **180;** relaxing the pressure of idler rollers **156, 731** on tubing **112;** restoring rotor **152, 730** to the start angle: re-establishing the pressure of idler rollers **156, 731** on tubing **112;** and opening valve **180.**

All of the embodiments of the rotary peristaltic pump of the present invention are characterized by the idler roller pressure on the tube bearing the fluid being relieved within the pump while the rotor is returned to its start angle after the dispensing portion of the dispensing cycle is completed. This is achieved by one of retracting the idler rollers of the rotor, moving the stator to a location distant from the rotor, or moving the rotor to a location distant from the stator. The phrase "determined amount of fluid" is used in the present disclosure to describe either or both of an amount of fluid measured during the dispensing of fluid via dispensing needle **130** of **FIG. 1****,** and an amount of fluid determined based on an angle of rotation of rotor **152, 730** of pump **150, 150'.**

While this invention has been described as having an exemplary design, the present invention may be further modified within the spirit and scope of this disclosure. This application is therefore intended to cover any variations, uses, or adaptations of the invention using its general principles. Further, this application is intended to cover such departures from the present disclosure as come within known or customary practice in the art to which this invention pertains.

Examples of the present disclosure are set out in the following numbered clauses:
1. A peristaltic pump system comprising:
   a rotary peristaltic pump comprising a stator and a rotor, the rotor driven to rotate about a rotor axis and comprising a plurality of idler rollers arranged radially equidistant about the rotor axis, each roller freely rotating about an own axis parallel to the rotor axis;
   a flow valve in fluid communication with an output of the pump;
   a fluid path in fluid communication with the valve and comprising flexible tubing disposed between the plurality of rollers and the stator such that when the rotor is rotated at least one of the plurality of rollers may exert a pressure on the tubing against the stator;
   a controller in communication with the pump and with the valve, the controller comprising a processor and a memory: and
   software instructions which when loaded in the memory and executed by the processor effect at the end of a dispensing portion of a dispensing cycle in the following order closing of the valve, operating of the pump to relieve the pressure of the rollers on the tubing, and rotating of the rotor to a start angle.
2. The peristaltic pump system of clause 1, wherein:
   the pump further comprises a linear actuator arranged to reciprocate the stator between a first location proximate the rotor and a second location distant from the rotor: and
   the software instructions further including instructions which when loaded in the memory and executed by the processor effect to relieve the pressure of the rollers on the tubing cause the linear actuator to move the stator from the first location to the second location.
3. The peristaltic pump system of clause 1, wherein:
   the pump further comprises a linear actuator arranged to reciprocate the rotor between a first location proximate the stator and a second location distant from the stator; and
   the software instructions further including instructions which when loaded in the memory and executed by the processor effect to relieve the pressure of the rollers on the tubing cause the linear actuator to move the rotor from the first location to the second location.
4. The peristaltic pump system of clause 1, wherein:
   the plurality of rollers are retractable into the rotor; and
   the software instructions further including instructions which when loaded in the memory and executed by the processor effect to relieve the pressure of the plurality of rollers on the tubing by causing the rollers to be retracted.
5. The peristaltic pump system of clause 1, wherein:
   the pump further comprises a linear actuator arranged to reciprocate the plurality of rollers between a first location extending beyond the rotor proximate the stator and a second location distant from the stator: and
   the software instructions further including instructions which when loaded in the memory and executed by the processor effect to relieve the pressure of the plurality of rollers on the tubing by causing the rollers to be retracted to the second location.
6. A method for advancing a determined amount of fluid from a fluid source through a flow valve, the method comprising:
   providing the fluid source, the flow valve, a rotary peristaltic pump, a controller configured to control the pump and the valve, and a fluid path placing the fluid source in fluid communication with the flow valve through the pump, the pump comprising: a stator; a rotor driven to rotate about a rotor axis, the rotor comprising a plurality of idler rollers arranged radially equidistant about the rotor axis, each roller freely rotating about an own axis parallel to the rotor axis, wherein the fluid path comprises flexible tubing disposed between the rollers and the stator;
   exerting pressure on the flexible tubing with the idler rollers when the valve is open, and rotating the rotor from a start angle to a dispense angle to advance the determined amount of fluid from the fluid source through the valve;
   closing the valve after advancing the determined amount of fluid;
   relaxing the pressure of the plurality of idler rollers on the tubing after closing the valve;
   when the pressure on the tubing is relaxed and the valve closed, restoring the rotor to the start angle;
   re-establishing the pressure of the plurality of idler rollers on the tubing; and
   opening the valve after re-establishing the pressure of the plurality of idler rollers on the tubing.
7. The method of clause 6, wherein:
   providing the rotary peristaltic pump comprises providing the peristaltic pump with the stator arranged to reciprocate between a first location proximate the rotor and a second location distant from the rotor; and wherein
   relaxing the pressure of the plurality of idler rollers on the tubing comprises moving the stator from the first location to the second location.
8. The method of clause 6, wherein:
   providing the rotary peristaltic pump comprises providing the peristaltic pump with the rotor arranged to reciprocate between a first location proximate the stator and a second location distant from the stator: and wherein
   relaxing the pressure of the idler rollers on the tubing comprises moving the rotor from the first location to the second location.
9. The method of clause 6, wherein:
   providing the rotary peristaltic pump comprises providing the peristaltic pump with the idler rollers arranged to be retractable into the rotor; and wherein
   relaxing the pressure of the idler rollers on the tubing comprises retracting the rollers into the rotor.
10. The method of clause 6, wherein the step of relaxing the pressure of the plurality of rollers involves physically removing the plurality of rollers from contacting the tubing.
11. A method for aseptically filling a container with a pharmaceutical fluid, the method comprising:
   providing a fluid source, a fill needle and container disposed within a sterile isolator, a flow valve, a rotary peristaltic pump, a controller configured to control the pump and the valve, and a fluid path extending between the fluid source and the flow valve and from the valve to the fill needle disposed above an opening of a first container, the pump comprising: a stator; a rotor driven to rotate about a rotor axis, the rotor comprising a plurality of idler rollers arranged radially equidistant about the rotor axis, each roller freely rotating about an own axis parallel to the rotor axis, wherein the fluid path comprises flexible tubing disposed between the rollers and the stator;
   moving at least one of the container and the fill needle to locate the fill needle over an opening in the container;
   opening the valve and exerting pressure on the flexible tubing with the idler rollers by rotating the rotor from a start angle to a dispense angle to advance the determined amount of fluid from the fluid source through the valve and the fill needle into the container;
   closing the valve after advancing the fluid;
   relaxing the pressure of the idler rollers on the tubing after closing the valve:
   when the pressure on the tubing is relaxed and the valve is closed, restoring the rotor to the start angle;
   re-establishing the pressure of the idler rollers on the tubing; and
   opening the valve after re-establishing the pressure of the idler rollers on the tubing.
12. The method of clause 11, wherein:
   providing the rotary peristaltic pump comprises providing the peristaltic pump with the stator arranged to reciprocate between a first location proximate the rotor and a second location distant from the rotor; and wherein
   relaxing the pressure of the idler rollers on the tubing comprises moving the stator from the first location to the second location.
13. The method of clause 11, wherein:
   providing the rotary peristaltic pump comprises providing the peristaltic pump with the rotor arranged to reciprocate between a first location proximate the stator and a second location distant from the stator; and wherein
   relaxing the pressure of the idler rollers on the tubing comprises moving the rotor from the first location to the second location.
14. The method of clause 11, wherein:
   providing the rotary peristaltic pump comprises providing the peristaltic pump with the idler rollers arranged to be retractable into the rotor; and wherein
   relaxing the pressure of the idler rollers on the tubing comprises retracting the rollers into the rotor.
15. The method of clause 11, wherein the step of relaxing the pressure of the plurality of rollers involves physically removing the plurality of rollers from contacting the tubing.
16. A method for aseptically filling a plurality of containers with a pharmaceutical fluid, the method comprising:
   (a) providing a fluid source, disposed within a sterile isolator a fill needle and the plurality of containers within a container nest, a flow valve, a rotary peristaltic pump, a controller configured to control the pump and the valve, and a fluid path extending between the fluid source and the flow valve and from the valve to the fill needle disposed above an opening of a first container, the pump comprising: a stator; a rotor driven to rotate about a rotor axis, the rotor comprising a plurality of idler rollers arranged radially equidistant about the rotor axis, each roller freely rotating about an own axis parallel to the rotor axis, wherein the fluid path comprises flexible tubing disposed between the rollers and the stator;
   (b) moving at least one of the container nest and the fill needle to locate the fill needle over an opening of a first of the plurality of containers;
   (c) when the idler rollers are exerting pressure on the flexible tubing and the valve is open, rotating the rotor from a start angle to a dispense angle to advance the determined amount of fluid from the fluid source through the valve and the fill needle into the first of the plurality of containers;
   (d) closing the valve after advancing the fluid;
   (e) relaxing the pressure of the idler rollers on the tubing after closing the valve:
   (f) when the pressure on the tubing is relaxed and the valve is closed restoring the rotor to the start angle;
   (g) re-establishing the pressure of the idler rollers on the tubing;
   (h) moving at least one of the container nest and the fill needle to locate the fill needle over an opening of another of the plurality of containers; and
   (i) opening the valve;
   (j) repeating steps (c) to (i) until the plurality of containers have been filled with fluid.
17. The method of clause 16, wherein:
   providing the rotary peristaltic pump comprises providing the peristaltic pump with the stator arranged to reciprocate between a first location proximate the rotor and a second location distant from the rotor: and wherein
   relaxing the pressure of the idler rollers on the tubing comprises moving the stator from the first location to the second location.
18. The method of clause 16, wherein:
   providing the rotary peristaltic pump comprises providing the peristaltic pump with the rotor arranged to reciprocate between a first location proximate the stator and a second location distant from the stator; and wherein
   relaxing the pressure of the idler rollers on the tubing comprises moving the rotor from the first location to the second location.
19. The method of clause 16, wherein:
   providing the rotary peristaltic pump comprises providing the peristaltic pump with the idler rollers arranged to be retractable into the rotor; and wherein
   relaxing the pressure of the idler rollers on the tubing comprises retracting the rollers into the rotor.
20. The method of clause 16, wherein the step of relaxing the pressure of the plurality of rollers involves physically removing the plurality of rollers from contacting the tubing.

## Claims

1. A rotary peristaltic pump (150) comprising a stator (154) and a rotor (152), the rotor (152) driven to rotate about a rotor axis (153) and comprising a plurality of idler rollers (156) arranged radially equidistant about the rotor axis (153), each roller (156) freely rotating about an own axis (157) parallel to the rotor axis (153), said pump configured to exert pressure on a tubing (112) via the plurality of idler rollers (156).

2. The pump according to claim 1, further comprising a controller (170) in communication with the pump (150) and configured for being in communication with a valve (180), the controller (170) comprising a processor, a memory, and software instructions which, when loaded in the memory and executed by the processor, effect at the end of a dispensing portion of a dispensing cycle in the following order:
closing the valve (180);
operating the pump (150) to relieve the pressure of at least one of the plurality of rollers (156) on the tubing (112); and
rotating of the rotor (152) to a start angle.

3. The pump according to claim 2, further comprising a linear actuator (158) arranged to reciprocate the stator (154) between a first location proximate the rotor (152) and a second location distant from the rotor (152); and the software instructions further including instructions which when loaded in the memory and executed by the processor effect to relieve the pressure of the at least one of the plurality of rollers on the tubing by causing the linear actuator to move the stator from the first location to the second location.

4. The pump according to claim 2, further comprising a linear actuator (158) arranged to reciprocate the rotor (152) between a first location proximate to the stator (154) and a second location distant from the stator (154); and the software instructions further including instructions which when loaded in the memory and executed by the processor effect to relieve the pressure of the at least one of the plurality of rollers (156) on the tubing (112) by causing the linear actuator to move the rotor from the first location to the second location.

5. The pump according to claim 2, wherein:
the plurality of rollers (731) is retractable into the rotor; and
the software instructions further including instructions which when loaded in the memory and executed by the processor effect to relieve the pressure of the at least one of the plurality of rollers (731) on the tubing (112) by causing the rollers (731) to be retracted.

6. The pump according to claim 2, wherein:
the pump (150) further comprises a linear actuator (750) arranged to reciprocate the plurality of rollers (156, 731) between a first location extending beyond the rotor (152, 730) proximate the stator (154, 780) and a second location distant from the stator (780); and
the software instructions further including instructions which when loaded in the memory and executed by the processor effect to relieve the pressure of the at least one of the plurality of rollers (731) on the tubing (112) by causing the rollers to be retracted to the second location.

7. The pump according to claim 6, wherein each roller of the plurality of rollers (731) does not rotate about a fixed axis arranged about the rotor axis.

8. A controller (170) configured for being in communication with a pump (150) according to any of claims 1-7 and configured for being in communication with a valve (180), the controller (170) comprising a processor, a memory, and software instructions, said instructions as recited in any of claims 2-7.

9. A system (100) for aseptically filling a container (144) within a sterile isolator (140) with a pharmaceutical fluid (122) from fluid source container (120), said system comprising:
a fluid path (110);
the pump (150) according to any of claims 1-7;
a controller (170), said controller as recited in claim 8;
a valve (180); and
a fill needle (130);
wherein:
the pump and the valve are configured to be controlled by the controller;
the fluid path is configured to enter the sterile isolator via a port (142); and
the fluid path within the pump is defined by a flexible tube (112).

10. The system according to claim 9, wherein:
the pump is configured to be controlled by the controller via a pump control line (160); and
the valve is configured to be controlled by the controller via a valve control line (190).

11. The system according to claim 9, wherein:
the pump and the valve are configured to be controlled by the controller via a telecommunications protocol, optionally wherein the telecommunications protocol is Bluetooth or Wi-Fi.

12. The system according to any of claims 9-11, further comprising a further mechanism for reciprocating the rotor between a first and a second rotor location, said further mechanism comprising a rotor configured to be the only movable portion of the pump.

13. The system according to any of claims 9-12, wherein a memory comprised in the controller further comprises instructions configured, when executed by a processor, to:
move a rotor (152) of the pump to a first location;
open the valve, and then rotate the rotor from a start angle to a dispense angle to allow a determined amount of fluid to flow from the fluid source along the flow path through the flexible tube and through the valve;
subsequent to rotating the rotor to the dispense angle, close the valve;
relax pressure on the flexible tube by moving the rotor to a second location, and then restore the rotor to the start angle without causing the fluid to flow through the valve; and
optionally, repeating the steps recited above.

14. The use of the system according to any of claims 9-13 for filling a container (144).

15. A non-transitory computer-readable medium comprising processor-executable instructions as recited in any of claims 2-7 or 13.
